# EUROPEAN PATENT SPECIFICATION

(11) **EP 3 684 250 B1**
(45) Date of publication and mention of the grant of the patent: **19.07.2023**
(21) Application number: 18859773.6
(22) Date of filing: 14.09.2018
(51) Int. Cl.: A61B 5/022, A61B 5/021, A61B 5/00, A61B 5/0225

(54) **FINGER CUFF FOR AND METHOD OF BLOOD PRESSURE MEASURING**
FINGERMANSCHETTE FÜR UND VERFAHREN ZUR BLUTDRUCKMESSUNG
MANCHON DE DOIGT POUR ET MÉTHODE DE MESURE DE LA PRESSION SANGUINE

(30) Priority: 19.09.2017 US 201762560415 P; 12.09.2018 US 201816129619
(43) Date of publication of application: 29.07.2020
(73) Proprietor: Edwards Lifesciences Corporation, Irvine, CA 92614 (US)
(72) Inventor: LI, Peiyuan, Irvine, CA 92614 (US); SCHRAA, Olaf, Irvine, CA 92614 (US); VAN DER SAR, Geertruida, Lucretia, Irvine, CA 92614 (US)
(74) Representative: Eisenführ Speiser
(86) International application number: PCT/US2018/051016
(87) International publication number: WO 2019/060212

(56) References cited:
- WO-A1-01/22868
- WO-A1-2016/146356
- CN-A- 105 030 195
- CN-U- 202 723 841
- US-A- 6 120 459
- US-A1- 2004 181 254
- US-A1- 2012 238 887
- US-A1- 2013 190 576
- DING XIAO-RONG ET AL: "Continuous Blood Pressure Measurement From Invasive to Unobtrusive: Celebration of 200th Birth Anniversary of Carl Ludwig", IEEE JOURNAL OF BIOMEDICAL AND HEALTH INFORMATICS, IEEE, PISCATAWAY, NJ, USA, vol. 20, no. 6, 1 November 2016 (2016-11-01), pages 1455-1465, XP011635972, ISSN: 2168-2194, DOI: 10.1109/JBHI.2016.2620995 [retrieved on 2016-12-06]

## Description

### BACKGROUND

### Field

Embodiments of the invention relate generally to non-invasive blood pressure measurement. More particularly, embodiments of the invention relate to a finger cuff to apply concentrated pressure to the bottom (e.g., 'bottom' side denotes here the palmar side) of a patient's finger.

### Relevant Background

Volume clamping is a technique for non-invasively measuring blood pressure in which an external pressure is applied to a patient's finger in such a manner that arterial pressure may be balanced by a time varying pressure to maintain a constant arterial volume. In a properly fitted and calibrated system, the applied time varying pressure is equal to the arterial blood pressure in the finger. The applied time varying pressure may be measured to provide a reading of the patient's arterial blood pressure.

This may be accomplished by a finger cuff that is arranged around a finger of a patient. The finger cuff may include an infrared light source, an infrared sensor, and an inflatable bladder. The infrared light may be sent through the finger in which a finger artery is present. The infrared sensor picks up the infrared light and the amount of infrared light registered by the sensor may be inversely proportional to the artery diameter and indicative of the pressure in the artery.

In the finger cuff implementation, by inflating the bladder of the finger cuff, a pressure is exerted on the finger artery. If the pressure is high enough, it will compress the artery and the amount of light registered by the sensor will increase. The amount of pressure necessary in the inflatable bladder to compress the artery is dependent on the blood pressure. By controlling the pressure of the inflatable bladder such that the diameter of the finger artery is kept constant, the blood pressure may be monitored in very precise detail as the pressure in the inflatable bladder is directly linked to the blood pressure. In a typical present day finger cuff implementation, a volume clamp system is used with the finger cuff. The volume clamp system typically includes a pressure generating system and a regulating system that includes: a pump, a valve, and a pressure sensor in a closed loop feedback system that are used in the measurement of the arterial volume. To accurately measure blood pressure, the feedback loop provides sufficient pressure generating and releasing capabilities to match the pressure oscillations of the patient's blood pressure.

Today, finger cuffs typically use an air bladder to apply pressure on the whole circumference of the finger. When the finger cuff is applied on the patient's finger, and the air bladder is inflated during use for blood pressure measurement, the finger is pressurized around its whole circumference.
WO 01/22868 A1 describes a finger holder for attaching to the patient's finger and an optical measurement device utilizing the finger holder for performing non-invasive measurements of patient's blood parameters. The finger holder comprises a first member and second member spaced apart from each other. The first member is designed to secure a fingertip and to support a measuring unit mounted so as to apply optical measurements to a first location of the finger. The second member is associated with a pressurizing assembly mounted to apply desired over-systolic pressure to a second location on the patient' s finger upstream of the first location with respect to a normal blood flow direction. A substantially rigid connector connects the first and second members to each other, and is designed to engage the finger along its middle phalanx and proximal interphalangeal joint, thereby preventing it from folding during the measurements, when the over-systolic pressure is applied to the finger. WO 2016/146356 A1 describes an apparatus for measuring the blood pressure, BP, of a user, the apparatus comprising a volume-clamp BP monitoring device that comprises a first pressure device for applying pressure to a first part of the body of the user, a first photoplethysmogram, PPG, sensor for obtaining a first PPG signal from the first part of the body of the user, and a control unit that is configured to analyze the first PPG signal and to control the pressure of the first pressure device. The control unit is configured to adjust the pressure of the first pressure device to maintain the first PPG signal at a constant level and to determine the BP of the user from the pressure of the first pressure device. A second sensor, separate from the first PPG sensor, is provided for measuring a physiological characteristic of the user in a second part of the body of the user. The second part of the body is separate from the first part of the body. The apparatus is configured to analyze the measured physiological characteristic to determine a measure of the blood perfusion in the second part of the body of the user, and to determine whether to perform a recalibration of the volume-clamp BP monitoring device on the basis of changes in the blood perfusion. CN 202723841 U describes a finger cuff in accordance with the preamble of claim 1.

It may be beneficial to utilize a bladder in a finger cuff that uses one or more partial bladders to implement different functions.

### SUMMARY

Embodiments of the invention relate to a finger cuff that is applied on a patient's finger to be used in measuring the patient's blood pressure by a blood pressure measurement system utilizing the volume clamp method, in accordance with claim 1 and a method to measure a patient's blood pressure according to claim 6.

### BRIEF DESCRIPTION OF THE DRAWINGS

FIG. 1 is a diagram of an example of a blood pressure measurement system.
FIG. 2 is a diagram illustrating an example of a finger cuff according to one embodiment.
FIGs. 3A-3B are diagrams illustrating cross-sectional views of the finger cuff with differing bladders attached to a patient's finger fig. 3B is according to one embodiment.
FIG. 4 is a block diagram illustrating an example environment in which embodiments of the invention may be practiced.

### DETAILED DESCRIPTION

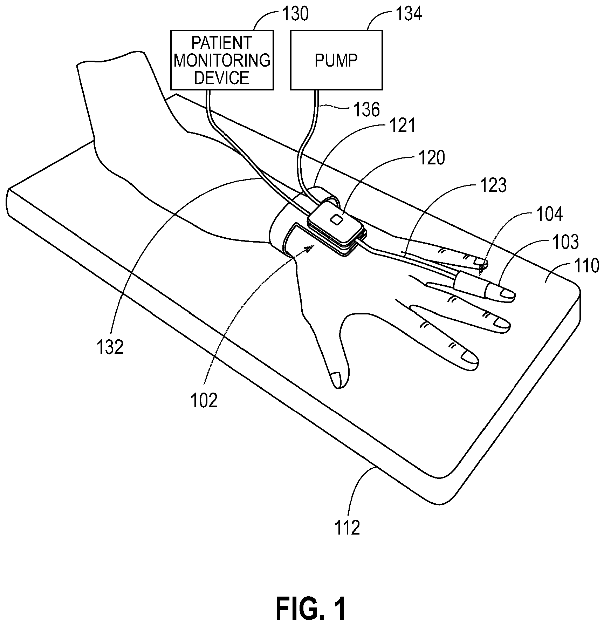

With reference to Figure 1, which illustrates an example of a blood pressure measurement system 102 that includes a finger cuff 104 that may be applied to a patient's finger 103 and a blood pressure measurement controller 120, which may be attached to the patient's body (e.g., a patient's wrist or hand).

The blood pressure measurement system 102 may further be connected to a patient monitoring device 130, and, in some embodiments, a pump 134. Further, finger cuff 104 may include a bladder (not shown) and an LED-PD pair (not shown), which are conventional for finger cuffs.

The blood pressure measurement system 102 may include a pressure measurement controller 120 that includes: a small internal pump, a small internal valve, a pressure sensor, and control circuity. In this embodiment, the control circuitry may be configured to: control the pneumatic pressure applied by the internal pump to the bladder of the finger cuff 104 to replicate the patient's blood pressure based upon measuring the plethysmogram signal received from the LED-PD pair of the finger cuff 104. Further, the control circuitry may be configured to:
control the opening of the internal valve to release pneumatic pressure from the bladder; or the internal valve may simply be an orifice that is not controlled.

Additionally, the control circuitry may be configured to: measure the patient's blood pressure by monitoring the pressure of the bladder based upon the input from a pressure senor, which should be the same as patient's blood pressure, and may display the patient's blood pressure on the patient monitoring device 130.

A conventional pressure generating and regulating system may be utilized, in which, a pump 134 is located remotely from the body of the patient. In this embodiment, the blood pressure measurement controller 120 receives pneumatic pressure from remote pump 134 through tube 136 and passes on the pneumatic pressure through tube 123 to the bladder of finger cuff 104. Blood pressure measurement device controller 120 may also control the pneumatic pressure (e.g., utilizing a controllable valve) applied to the finger cuff 104 as well as other functions. In this example, the pneumatic pressure applied by the pump 134 to the bladder of finger cuff 104 to replicate the patient's blood pressure based upon measuring the plethysmogram signal received from the LED-PD pair of the finger cuff 104 (e.g., to keep the plethysmogram signal constant) and measuring the patient's blood pressure by monitoring the pressure of the bladder may be controlled by the blood pressure measurement controller 120 and/or a remote computing device and/or the pump 134 and/or the patient monitoring device 130 to implement the volume clamping method. In some embodiments, a blood pressure measurement controller 120 is not used at all and there is simply a connection from tube 136 from a remote pump 134 including a remote pressure regulatory system to finger cuff 104, and all processing for the pressure generating and regulatory system, data processing, and display is performed by a remote computing device.

Continuing with this example, as shown in Figure 1, a patient's hand may be placed on the face 110 of an arm rest 112 for measuring a patient's blood pressure with the blood pressure measurement system 102. The blood pressure measurement controller 120 of the blood pressure measurement system 102 may be coupled to a bladder of the finger cuff 104 in order to provide pneumatic pressure to the bladder for use in blood pressure measurement. Blood pressure measurement controller 120 may be coupled to the patient monitoring device 130 through a power/data cable 132. Also, in one embodiment, as previously described, in a remote implementation, blood pressure measurement controller 120 may be coupled to a remote pump 134 through tube 136 to receive pneumatic pressure for the bladder of the finger cuff 104. The patient monitoring device 130 may be any type of medical electronic device that may read, collect, process, display, etc., physiological readings/data of a patient including blood pressure, as well as any other suitable physiological patient readings. Accordingly, power/data cable 132 may transmit data to and from patient monitoring device 130 and also may provide power from the patient monitoring device 130 to the blood pressure measurement controller 120 and finger cuff 104.

As can be seen in Figure 1, in one example, the finger cuff 104 may be applied to a patient's finger 103 and the blood pressure measurement controller 120 may be attached on the patient's hand or wrist with an attachment bracelet 121 that wraps around the patient's wrist or hand. The attachment bracelet 121 may be metal, plastic, Velcro, etc. It should be appreciated that this is just one example of attaching a blood pressure measurement controller 120 and that any suitable way of attaching a blood pressure measurement controller to a patient's body or in close proximity to a patient's body may be utilized and that, in some embodiments, a blood pressure measurement controller 120 may not be used at all. It should further be appreciated that the finger cuff 104 may be connected to a blood pressure measurement controller described herein, or a pressure generating and regulating system of any other kind, such as a conventional pressure generating and regulating system that is located remotely from the body of the patient (e.g., a pump 134 located remotely from a patient). Any kind of pressure generating and regulating system can be used, including but not limited to the blood pressure measurement controller, and may be described simply as a pressure generating and regulating system that may be used with a finger cuff 104 including an LED-PD pair and a bladder to implement the volume clamping method.

Embodiments of the invention relate to applying concentrated pressure on the bottom of the patient's finger near the patient's arteries, instead of applying pressure all around the finger, as is the current practice. As will be described, this may be achieved by utilizing a bladder in a finger cuff that uses one or more partial bladders to implement different functions.

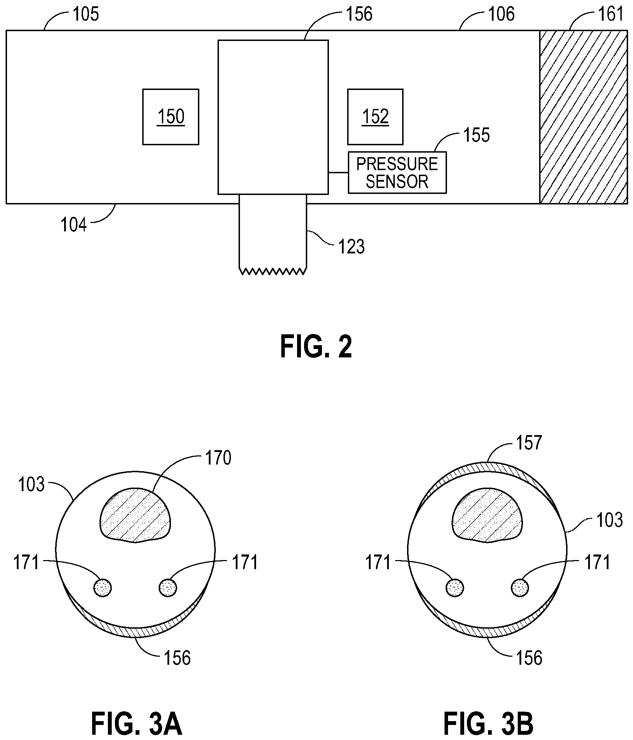

With additional reference to FIG. 2, in conjunction with the previously described FIG. 1, a finger cuff 104 that is connectable to a patient's finger 103, may be used in measuring the patient's blood pressure by a blood pressure measurement system 102 utilizing the volume clamp method. For example, the previously described blood pressure measurement system 102 may be utilized, or any suitable type of blood pressure measurement system.

An example finger cuff 104, as shown in FIG. 2, will now be described. Finger cuff 104 may include a first side 105 and a second side 106. In one example, for attachment purposes to a patient's finger 103, the second side 106 of the interior may have a first Velcro type portion 161 that connects with a second Velcro type portion on the exterior of the first side 105 of the finger cuff 104. It should be appreciated that this is just one example of an attachment mechanism and that any suitable type may be utilized (e.g., adhesive, tape, etc.). It should also be appreciated that any type of wrappable finger cuff, fixed type of finger cuff, or any type of finger cuff may be utilized, and this is just one example.

Further, finger cuff 104 may have a bladder 156 and an LED-PD pair 150 and 152. The LED-PD pair 150 and 152 may be used to perform measurements of a plethysmogram signal to aid in measuring the patient's blood pressure. Also, tube 123 may be connected to bladder 156 to apply pneumatic pressure to bladder 156.

In particular, as shown in FIG. 2, bladder 15 includes a bottom portion that is denoted bottom portion bladder 156 that is sized to abut only the bottom portion of the patient's finger 103 to apply concentrated pressure only on the bottom of the patient's finger 103 near both of the patient's two arteries, wherein, when the finger cuff 104 is placed around the patient's finger 103, the bottom portion bladder 156 and the LED-PD pair 150 and 152 aid in measuring the patient's blood pressure by the blood pressure measurement system using the volume clamp method. As an example, as part of the volume clamp method, pneumatic pressure is applied to the bottom portion bladder 156 of the finger cuff 104 based upon measuring the plethysmogram signal received from the LED-PD pair 150 and 152 of the finger cuff 104 (e.g., to keep the plethysmogram signal constant) so that the pressure applied to the bottom portion bladder 156 and measured by the pressure sensor 155 should be correlated to the patient's blood pressure. It should be appreciated that the pressure sensor 155 can be connected to the bladder or tube at various locations. In particular, applying concentrated pressure on the bottom of the patient's finger near the arteries, by the bottom portion bladder 156, instead of applying pressure all around the finger, as is the current practice, provides many benefits, as will be described. Thus, in this configuration, a partial bottom portion bladder 156 is utilized. Further, an additional partial bladder is also used for additional functions, as will be described.

With additional reference to FIGs. 3A and 3B, showing cross sections, a more particular description of embodiments of the invention will be described. With particular additional reference to FIG. 3A, the bottom portion bladder 156 abuts only the bottom portion of the patient's finger 105 to apply concentrated pressure only to the bottom of the patient's finger 103 near both of the patient's two arteries 171 and 171. The patient's bone 170 is also shown. Therefore, when the finger cuff 104 is placed around the patient's finger 103, the bottom portion bladder 156 and the LED-PD pair 150 and 152 may be used to aid in measuring the patient's blood pressure by the blood pressure measuring system and the volume clamp method, as has been described.

Therefore, in this implementation, the bottom portion bladder 156 forms only one pressure area where pressure is applied directly under both of the patient's two fingers arteries 171 and 171. Also, as has been described, the pressure sensor 155 may be coupled to the bottom portion bladder 156 to measure the air pressure of the bottom portion bladder 156 to aid in determining the patient's blood pressure as part of the volume clamp method by the blood pressure measurement system. As an example, as part of the volume clamp method, pneumatic pressure is applied to the bottom portion bladder 156 of the finger cuff 104 based upon measuring the plethysmogram signal received from the LED-PD pair 150 and 152 of the finger cuff 104 (e.g., to keep the plethysmogram signal constant) so that the pressure applied to the bottom portion bladder 156 and measured by the pressure sensor 155 should be correlated to the patient's blood pressure.

As can be particularly seen, in FIG. 3A, the bladder including a particular bottom portion bladder 156 applies concentrated pressure only on the bottom of the patient's finger 103 near the patient's two arteries 171 and 171. In this way, only one pressure area is applied directly under patient's two arteries 171 and 171. Therefore, this singular bottom portion bladder 156 can be used to apply pressure and for measurement purposes with the pressure sensor for use with the volume clamp method by the blood pressure measurement system.

With additional reference to FIG. 3B, the bladder configuration according to the invention further includes a second top portion bladder 157 for use in conjunction with the first bottom portion bladder 156 such that two different pressure areas on the patient's finger 103 may be utilized. Thus, the second top portion bladder 157 is on the opposite side of the first bottom portion bladder 156 and is on the top side of the patient's finger 103. As will be described, the second top portion bladder 157 may be used to compensate for variations in finger shape, finger size, and volume change due to pressure measurement through time.

Therefore, in one embodiment, the bottom portion bladder 156 may be used to apply pressure to the patient's arteries 171 and 171 of the patient's finger 103 for use in blood pressure measurement via the volume clamp method by the blood measurement system, as previously described. On the other hand, the second pressure area provided by the top portion bladder 157 on top of the patient's finger 103 on the opposite side of the first pressure area provided by the bottom portion bladder 156 for blood pressure measurement provides discrete and different features than the bottom portion bladder 156 (i.e., unrelated to blood pressure measurement). In particular, the second pressure area provided by the top portion bladder 157 can be used to compensate for variations in the patient's finger shape, finger size, and volume change (e.g., due to pressure measurement through time). Further, the top portion bladder 157 may be used to increase the comfort of the use of the finger cuff to the patient. Moreover, the second pressure area provided by the top portion bladder 157 may be used to stabilize the blood pressure measurement of the bottom portion bladder 156. For example, the top portion bladder 157 may be set to a constant pressure or a variable pressure that has been determined to optimize the accuracy of the blood pressure measurement in the volume clamp implementation that is reliant upon the performance of the bottom portion bladder 156.

Many benefits may be provided by the use of the bottom portion bladder 156 in conjunction with, the opposite top portion bladder 157. These benefits may include: improved measurement accuracy due to the concentrated pressure application of the bottom portion bladder 156; and simplified product design for the finger cuff. For example, the bottom portion bladder 156 itself is smaller and therefore less costly than current bladders for finger cuffs. Further, the accompanying pressure sensor 155 and other components of the finger cuff may also have a reduced size. Moreover, with this type of implementation, a one size fits all finger cuff may be implemented. In other words, separately sized finger cuffs (e.g., small, medium, large, etc.) for differently sized fingers may no longer be required because the finger cuff according to embodiments of the invention may be fitted to most any sized finger. In particular, the top portion bladder 157 may be adjusted in size to provide fitting to most any sized finger, such that the finger cuff may be one size fits all. Other benefits such as, ease of use, versatility (e.g., enabling other opportunities for product design), reduced product costs, increased comfort for the patient, are also provided.

Further, it should be appreciated that the pressure applied on the pressure areas by the bottom portion bladder 156 and the top portion bladder 157 may be generated by all types of medium - such as, air, liquid, electricity, etc. The previously described implementations can work with any of these types of mediums. Also, these types of pressure application areas utilizing differently sized bladder portions may be utilized with other extremity areas of the human body - such as, wrists, arms, ankles, legs, etc.

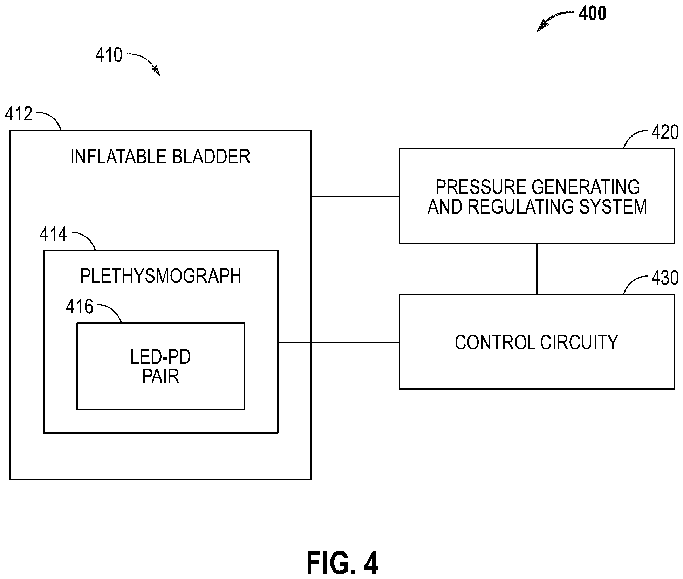

Figure 4 is a block diagram illustrating an example environment 400 in which embodiments of the invention may be practiced. As shown, finger cuff assembly 410 may include an inflatable bladder 412 and a plethysmograph 414. The inflatable bladder 412 may be pneumatically connected to a pressure generating and regulating system 420. The pressure generating and regulating system 420 may generate, measure, and regulate pneumatic pressure that inflates or deflates the inflatable bladder (e.g., the bottom portion bladder 156), and may include elements such as a pump, a valve, a sensor, control circuitry, and/or other suitable elements. When the bottom portion bladder 156 is inflated, a pressure is applied to the patient's finger. In particular, the volume clamp method is applied, in which, pneumatic pressure is applied to the bottom portion bladder 156 of the finger cuff based upon measuring the plethysmograph signal received from the LED-PD pair 416 (e.g., to keep the plethysmograph signal constant) so that the pressure applied to the bottom portion bladder 156 and measured by the pressure sensor should be correlated to the patient's blood pressure. The plethysmograph 414 may make continuous volumetric measurements (or plethysmogram) of arterial blood flows within the finger and, in one embodiment, may include the LED-PD pair 416. The LED may be used to illuminate the finger skin and light absorption or reflection may be detected with the photodiode. Therefore, the plethysmogram may be generated based on the signal received from the photodiode. The pressure generating and regulating system 420 and the plethysmograph 414 may be connected to a control circuitry 430. The control circuitry 430 may instruct the pressure generating and regulating system 420 to inflate or deflate the bottom portion bladder 156 and may receive data from the plethysmograph 414 and may carry out necessary data manipulations. Also, in some embodiments, the control circuitry 430 may instruct the pressure generating and regulating system 420 to inflate or deflate the top portion bladder 157.

It should be appreciated that aspects of the invention previously described may be implemented in conjunction with the execution of instructions by processors, circuitry, controllers, control circuitry, etc. As an example, control circuity may operate under the control of a program, algorithm, routine, or the execution of instructions to execute methods or processes in accordance with embodiments of the invention previously described. For example, such a program may be implemented in firmware or software (e.g. stored in memory and/or other locations) and may be implemented by processors, control circuitry, and/or other circuitry, these terms being utilized interchangeably. Further, it should be appreciated that the terms processor, microprocessor, circuitry, control circuitry, circuit board, controller, microcontroller, etc., refer to any type of logic or circuitry capable of executing logic, commands, instructions, software, firmware, functionality, etc., which may be utilized to execute embodiments of the invention.

The various illustrative logical blocks, processors, modules, and circuitry described in connection with the embodiments disclosed herein may be implemented or performed with a general purpose processor, a specialized processor, circuitry, a microcontroller, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A processor may be a microprocessor or any conventional processor, controller, microcontroller, circuitry, or state machine. A processor may also be implemented as a combination of computing devices, e.g., a combination of a DSP and a microprocessor, a plurality of microprocessors, one or more microprocessors in conjunction with a DSP core, or any other such configuration.

The steps of a method or algorithm described in connection with the embodiments disclosed herein may be embodied directly in hardware, in a software module/firmware executed by a processor, or any combination thereof. A software module may reside in RAM memory, flash memory, ROM memory, EPROM memory, EEPROM memory, registers, hard disk, a removable disk, a CD-ROM, or any other form of storage medium known in the art. An exemplary storage medium is coupled to the processor such the processor can read information from, and write information to, the storage medium. In the alternative, the storage medium may be integral to the processor.

The previous description of the disclosed embodiments is provided to enable any person skilled in the art to make or use the present invention.

## Claims

1. A finger cuff (104) attachable to a patient's finger (103) to be used in measuring the patient's blood pressure by a blood pressure measurement system utilizing the volume clamp method, the finger cuff (104) comprising:
a light emitting diode (150) and photodiode (152) to form an LED-PD pair to perform measurements of a plethysmogram signal to aid in measuring the patient's blood pressure; and
a bottom portion bladder (156) that abuts only a bottom portion of the patient's finger (103) to apply concentrated pressure only in a first pressure area formed by the bottom portion of the patient's finger under both of the patient's two arteries (171), wherein, when the finger cuff (104) is placed around the patient's finger (103), the bottom portion bladder (156) and LED-PD pair (150, 152) aid in measuring the patient's blood pressure by the blood pressure measurement system; wherein
a pressure sensor (155) coupled to the bottom portion bladder (156) is configured to measure the pressure of only the bottom portion bladder to aid in determining the patient's blood pressure as part of the volume clamp method by the blood pressure measurement system;
**characterized by**
a top portion bladder (157) on the opposite side of the bottom portion bladder (156), and configured to provide a second pressure area only on the top portion of the patient's finger.

2. The finger cuff of claim 1, further comprising a pressure generating and regulating system (420) pneumatically connected to the bottom portion bladder (156), and control circuitry (430) that is connected to the pressure generating and regulating system (420) and configured to instruct the pressure generating and regulating system (420) to inflate or deflate the bottom portion bladder (156).

3. The finger cuff of claim 2, wherein the control circuitry (430) is also configured to instruct the pressure generating and regulating system (420) to inflate or deflate the top portion bladder (157).

4. The finger cuff of claim 3, wherein the control circuitry (430) is configured to instruct the pressure generating and regulating system (420) to inflate or deflate the top portion bladder (157) to compensate for variations in finger shape, finger size, and volume change due to pressure measurement through time.

5. The finger cuff of claim 3, wherein the control circuitry (430) is configured to instruct the pressure generating and regulating system (420) to inflate or deflate the top portion bladder (157) to set it to a constant pressure or a variable pressure that has been determined to optimize an accuracy of the blood pressure measurement in the volume clamp implementation that is reliant upon the performance of the bottom portion bladder (156).

6. A method to measure a patient's blood pressure by a blood pressure measurement system utilizing a finger cuff (104) and the volume clamp method, the finger cuff (104) comprising a light emitting diode (LED) and photodiode to form an LED-PD pair (150, 152) to perform measurements of a plethysmogram signal to aid in measuring the patient's blood pressure and a bladder having a bottom portion (156), the method comprising:
inserting the patient's finger (103) into the finger cuff (104) such that a bottom bladder portion (156) abuts against only a bottom portion of the patient's finger; and
the bottom bladder portion (156) applying concentrated pressure only in a first pressure area formed by the bottom of the patient's finger (103), near both of the patient's two arteries (171);
the bottom portion bladder (156) and the LED-PD pair (150, 152) aiding in measuring the patient's blood pressure by the blood pressure measurement system; the method further comprising
a pressure sensor (155) coupled to the bottom portion bladder (156) measuring the pressure of only the bottom portion bladder to aid in determining the patient's blood pressure as part of the volume clamp method by the blood pressure measurement system;
**characterized by** the finger cuff having a top portion bladder (157) on the opposite side of the bottom portion bladder (156) providing a second pressure area on the top portion of the patient's finger.

7. The method of claim 6, wherein the finger cuff further comprises a pressure generating and regulating system (420) pneumatically connected to the bottom portion bladder (156); and using control circuitry (430) that is connected to the pressure generating and regulating system (420) to instruct the pressure generating and regulating system (420) to inflate or deflate the bottom portion bladder (156).

8. The method of claim 7, wherein the control circuitry (430) also instructs the pressure generating and regulating system (420) to inflate or deflate the top portion bladder (157).

9. The method of claim 8, wherein the control circuitry (430) instructs the pressure generating and regulating system (420) to inflate or deflate the top portion bladder (157) to compensate for variations in finger shape, finger size, and volume change due to pressure measurement through time.

10. The method of claim 7, wherein the control circuitry (430) instructs the
pressure generating and regulating system (420) to inflate or deflate the top portion bladder (157) to set it to a constant pressure or a variable pressure that has been determined to optimize an accuracy of the blood pressure measurement in the volume clamp implementation that is reliant upon the performance of the bottom portion bladder (156).

11. A blood pressure measurement system utilizing the volume clamp method, the blood pressure measurement system comprising a finger cuff according to any of the claims 1 to 5.

## Patentansprüche

1. Fingermanschette (104), die an einem Patientenfinger (103) zum Messen des Blutdrucks des Patienten mittels eines Blutdruckmesssystems unter Verwendung des Volume-Clamp-Verfahrens anbringbar ist, wobei die Fingermanschette (104) umfasst:
eine Licht emittierende Diode (150) und Photodiode (152), um ein LED-PD-Paar für die Unterstützung der Messung eines Plethysmogramm-Signals zu bilden, die Messung des Blutdrucks des Patienten zu bilden; und
eine Unterabschnittsblase (156), die nur an einem unteren Abschnitt des Patientenfingers (103) anliegt, um konzentrierten Druck nur in einem ersten Druckbereich, der durch den unteren Abschnitt des Patientenfingers unter den beiden Arterien des Patienten (171) gebildet ist, auszuüben, wobei, wenn die Fingermanschette (104) um den Patientenfinger (103) herum angebracht ist, die Unterabschnittsblase (156) und das LED-PD Paar (150, 152) die Messung des Blutdrucks des Patienten durch das Blutdruckmesssystem unterstützen; wobei
ein Drucksensor (155), der mit der Unterabschnittsblase (156) verbunden ist, ausgestaltet ist, den Druck ausschließlich in der Unterabschnittsblase zu messen, um die Bestimmung des Blutdrucks des Patienten als Teil des Volume-Clamp-Verfahrens durch das Blutdruckmesssystem zu unterstützen;
**gekennzeichnet durch**
eine Oberabschnittsblase (157) auf der gegenüberliegenden Seite der Unterabschnittsblase (156), die ausgestaltet ist, einen zweiten Druckbereich nur am oberen Abschnitt des Patientenfingers bereitzustellen.

2. Fingermanschette nach Anspruch 1, weiterhin umfassend ein Druckerzeugungs- und Druckregulierungssystem (420), das pneumatisch mit der Unterabschnittsblase (156) verbunden ist, sowie einen Regelkreis (430), der mit Druckerzeugungs- und Druckregulierungssystem (420) verbunden und ausgestaltet ist, das Druckerzeugungs- und Druckregulierungssystem (420) zum Füllen oder Ablassen der Unterabschnittsblase (156) zu instruieren.

3. Fingermanschette nach Anspruch 2, wobei der Regelkreis (420) weiterhin ausgebildet ist, das Druckerzeugungs- und Druckregulierungssystem (420) zum Füllen oder Ablassen der Oberabschnittsblase (157) zu instruieren.

4. Fingermanschette nach Anspruch 3, wobei der Regelkreis (430) ausgestaltet ist, das Druckerzeugungs- und Druckregulierungssystem (420) zum Füllen oder Ablassen der Oberabschnittsblase (157) zu instruieren, um Variationen der Fingerform, Fingergröße und Volumenänderung aufgrund der Druckmessung über die Zeit zu kompensieren.

5. Fingermanschette nach Anspruch 3, wobei der Regelkreis (430) ausgestaltet ist, das Druckerzeugungs- und Druckregulierungssystem (420) zum Füllen oder Ablassen der Oberabschnittsblase (157) zu instruieren, um in dieser einen konstanten Druck oder einen variablen Druck einzustellen, der bestimmt wurde, um die Genauigkeit des Blutdruckmesssystems in der Volume-Clamp Implementierung zu optimieren, die von den Leistungsmerkmalen der Unterabschnittsblase (156) abhängt.

6. Verfahren zur Messung des Blutdrucks eines Patienten durch ein Blutdruckmesssystem unter Verwendung einer Fingermanschette (104) und des Volume-Clamp-Verfahrens, wobei die Fingermanschette (104) umfasst:
eine Licht emittierende Diode (LED) und eine Photodiode zur Bildung eine LED-PD Paars (150, 152) zur Durchführung von Messungen eines Plethysmogramm-Signals, um die Messung des Blutdrucks des Patienten zu unterstützen, und einer Blase mit einem unteren Abschnitt (156); wobei das Verfahren umfasst:
Einführen des Patientenfingers (103) in die Fingermanschette (104) in einer Weise, dass eine Unterabschnittsblase (156) nur an einem unteren Abschnitt des Patientenfingers anliegt; und
die Unterabschnittsblase (156) konzentriert Druck nur in einem ersten Druckbereich, der durch die Unterseite des Patientenfingers (103) nahe den beiden Arterien des Patienten (171) begrenzt ist, ausübt;
die Unterabschnittsblase (156) und das LED-PD Paar (150, 152) bei der Messung des Blutdrucks des Patienten durch das Blutdruckmesssystem unterstützen; das Verfahren weiterhin umfasst:
einen mit der Unterabschnittsblase (156) verbundenen Drucksensor (155), der den Druck der Unterabschnittsblase misst, um die Bestimmung des Blutdrucks des Patienten als Teil des Volume-Clamp-Verfahrens durch das Blutdruckmesssystem zu unterstützen;
**gekennzeichnet durch**, dass
die Fingermanschette eine Oberabschnittsblase (157) auf der gegenüberliegenden Seite der Unterabschnittsblase (156) aufweist, die einen zweiten Druckbereich am oberen Abschnitt des Patientenfingers bereitstellt.

7. Verfahren nach Anspruch 6, wobei die Fingermanschette weiterhin umfasst:
ein Druckerzeugungs- und Druckregulierungssystem (420), das pneumatisch mit der Unterabschnittsblase (156) verbunden ist; und
Verwenden eines Regelkreises (430), der mit dem Druckerzeugungs- und Druckregulierungssystem (420) verbunden ist, um das Druckerzeugungs- und Druckregulierungssystem (420) zum Füllen oder Ablassen der Unterabschnittsblase (156) zu instruieren.

8. Verfahren nach Anspruch 7, wobei der Regelkreis (430) weiterhin das Druckerzeugungs- und Druckregulierungssystem (420) zum Füllen oder Ablassen der Oberabschnittsblase (157) instruiert.

9. Verfahren nach Anspruch 8, wobei der Regelkreis (430) das Druckerzeugungs- und Druckregulierungssystem (420) zum Füllen oder Ablassen der Oberabschnittsblase (157) instruiert, um Variationen der Fingerform, Fingergröße und Volumenänderung infolge der Druckmessung über die Zeit zu kompensieren.

10. Verfahren nach Anspruch 7, wobei der Regelkreis (430) das Druckerzeugungs- und Druckregulierungssystem (420) zum Füllen oder Ablassen der Oberabschnittsblase (157) instruiert, um in dieser einen konstanten Druck oder einen variablen Druck einzustellen, der bestimmt wurde, um die Genauigkeit des Blutdruckmesssystems infolge der Volume-Clamp Implementierung zu optimieren, die von den Leistungsmerkmalen der Unterabschnittsblase (156) abhängt.

11. Blutdruckmesssystem auf Basis des Volume-Clamp-Verfahrens, wobei das Blutdruckmesssystem eine Fingermanschette nach einem der Ansprüche 1 bis 5 umfasst.

## Revendications

1. Manchon de doigt (104) pouvant être fixé au doigt d'un patient (103) pour être utilisée dans la mesure de la tension artérielle du patient par un système de mesure de la tension artérielle utilisant le procédé de serrage volumique, manchon de doigt (104) comprenant :
une diode électroluminescente (light emitting diode, LED) (150) et une photodiode (152) pour former une paire LED-PD afin d'effectuer des mesures d'un signal de pléthysmogramme pour aider à mesurer la pression artérielle du patient ; et
une partie inférieure de vessie (156) qui vient en butée uniquement sur la partie inférieure du doigt du patient (103) pour appliquer une pression concentrée uniquement dans une première zone de pression formée par la partie inférieure du doigt du patient sous les deux artères du patient (171), dans laquelle, lorsque le manchon de doigt (104) est placé autour du doigt du patient (103), la partie inférieure de la vessie (156) et la paire LED-PD (150, 152) aident à mesurer la pression artérielle du patient par le système de mesure de la pression artérielle ; dans lequel
un capteur de pression (155) couplé à la partie inférieure de la vessie (156) est configuré pour mesurer la pression de la partie inférieure de la vessie uniquement afin d'aider à déterminer la pression artérielle du patient dans le cadre du procédé de serrage volumique par le système de mesure de la pression artérielle ;
**caractérisé par**
une partie supérieure de la vessie (157) située du côté opposé à la partie inférieure de la vessie (156) et configurée pour fournir une deuxième zone de pression uniquement sur la partie supérieure du doigt du patient.

2. Manchon de doigt selon la revendication 1, comprenant en outre un système de génération et de régulation de la pression (420) relié pneumatiquement à la partie inférieure de la vessie (156), et un circuit de commande (430) relié au système de génération et de régulation de la pression (420) et configuré pour ordonner au système de génération et de régulation de la pression (420) de gonfler ou de dégonfler la partie inférieure de la vessie (156).

3. Manchon de doigt selon la revendication 2, dans lequel le circuit de commande (430) est également configuré pour ordonner au système de génération et de régulation de la pression (420) de gonfler ou de dégonfler la partie supérieure de la vessie (157).

4. Manchon de doigt selon la revendication 3, dans lequel le circuit de commande (430) est configuré pour ordonner au système de génération et de régulation de la pression (420) de gonfler ou de dégonfler la partie supérieure de la vessie (157) afin de compenser les variations de la forme et de la taille du doigt et les changements de volume dus à la mesure de la pression dans le temps.

5. Manchon de doigt selon la revendication 3, dans lequel le circuit de commande (430) est configuré pour ordonner au système de génération et de régulation de la pression (420) de gonfler ou de dégonfler la partie supérieure de la vessie (157) pour la régler sur une pression constante ou une pression variable qui a été déterminée afin d'optimiser la précision de la mesure de la pression artérielle dans la mise en oeuvre du procédé de serrage volumique qui dépend de la performance de la partie inférieure de la vessie (156).

6. Procédé de mesure de la pression artérielle d'un patient par un système de mesure de la pression artérielle utilisant un manchon de doigt (104) et procédé de serrage volumique, le manchon de doigt (104) comprenant une diode électroluminescente (light emitting diode, LED) et une photodiode pour former une paire LED-PD (150, 152) afin d'effectuer des mesures d'un signal de pléthysmogramme pour aider à mesurer la pression artérielle du patient et une vessie ayant une partie inférieure (156), procédé comprenant :
l'insertion du doigt du patient (103) dans le manchon de doigt (104) de manière à ce qu'une partie inférieure de la vessie (156) soit en contact avec une partie inférieure du doigt du patient ; et
la partie inférieure de la vessie (156) appliquant une pression concentrée uniquement dans une première zone de pression formée par la partie inférieure du doigt du patient (103), à proximité des deux artères du patient (171) ;
la partie inférieure de la vessie (156) et la paire LED-PD (150, 152) aidant à mesurer la pression artérielle du patient par le système de mesure de la pression artérielle ; le procédé comprend en outre
un capteur de pression (155) couplé à la partie inférieure de la vessie (156) mesurant la pression de la partie inférieure de la vessie uniquement afin d'aider à déterminer la pression artérielle du patient dans le cadre du procédé de serrage volumique par le système de mesure de la pression artérielle ;
**caractérisé par**
le manchon de doigt ayant une partie supérieure de la vessie (157) située du côté opposé à la partie inférieure de la vessie (156) et fournissant une deuxième zone de pression uniquement sur la partie supérieure du doigt du patient.

7. Procédé selon la revendication 6, dans lequel le manchon de doigt comprend en outre un système de génération et de régulation de la pression (420) relié pneumatiquement à la partie inférieure de la vessie (156) ; et
utilisation d'un circuit de commande (430) connecté au système de génération et de régulation de la pression (420) pour ordonner au système de génération et de régulation de la pression (420) de gonfler ou de dégonfler la partie inférieure de la vessie (156).

8. Procédé selon la revendication 7, dans lequel le circuit de commande (430) ordonne également au système de génération et de régulation de la pression (420) de gonfler ou de dégonfler la partie supérieure de la vessie (157).

9. Procédé selon la revendication 8, dans lequel le circuit de commande (430) ordonne au système de génération et de régulation de la pression (420) de gonfler ou de dégonfler la partie supérieure de la vessie (157) afin de compenser les variations de la forme et de la taille du doigt et les changements de volume dus à la mesure de la pression dans le temps.

10. Procédé selon la revendication, dans lequel le circuit de commande (430) ordonne au système de génération et de régulation de la pression (420) de gonfler ou de dégonfler la partie supérieure de la vessie (157) pour la régler sur une pression constante ou une pression variable qui a été déterminée afin d'optimiser la précision de la mesure de la pression artérielle dans la mise en oeuvre du procédé de serrage volumique qui dépend de la performance de la partie inférieure de la vessie (156).

11. Système de mesure de la pression artérielle utilisant le procédé de serrage volumique, le système de mesure de la pression artérielle comprenant un manchon de doigt selon l'une des revendications 1 à 5.
